# EUROPEAN PATENT SPECIFICATION

(11) **EP 1 469 807 B1**
(45) Date of publication and mention of the grant of the patent: **16.03.2011**
(21) Application number: 03700642.6
(22) Date of filing: 08.01.2003
(51) Int. Cl.: A61F 13/62

(54) **DIAPER OR PANTS DIAPER**
WINDEL ODER HÖSCHENWINDEL
COUCHE OU COUCHE-CULOTTE

(30) Priority: 31.01.2002 SE 0200283
(43) Date of publication of application: 27.10.2004
(73) Proprietor: SCA Hygiene Products AB, 405 03 Göteborg (SE)
(72) Inventor: DAHLGREN, Marie, S-413 10 Göteborg (SE)
(74) Representative: Hyltner, Jan-Olof
(86) International application number: PCT/SE2003/000013
(87) International publication number: WO 2003/063749

(56) References cited:
- WO-A1-00/35399
- WO-A1-97/13485
- WO-A2-00/37009
- US-A- 5 722 127

## Description

### TECHNICAL AREA

The present invention relates to a diaper or pants diaper with a front section, a rear section, and an intermediate crutch section that has a narrower width than the front and rear sections, plus a longitudinal line of symmetry that extends from the middle of the rear edge of the rear section to the middle of the front edge of the front section, where the diaper or pants diaper includes an absorption body that is enclosed between an inner surface layer of material permeable to liquids and an outer surface layer of material impermeable to liquids, whereby the side parts of the front and rear sections are joinable to one another with a refastenable closure by means of a first mechanical join so that the diaper or pants diaper, in the position of use where the side parts are joined to one another in an over-lapping manner, acquires a pants-like configuration with one waist opening and two leg openings.

### BACKGROUND OF THE INVENTION

To join together the side parts of diapers and pants diapers, it is today common to use fasteners of the so-called Velcro^{®} type that extend from the waistline of the user over the hips. In this way, the diaper or pants diaper acquires a good fit, which is important for its function and that in addition gives an aesthetically pleasing appearance. The Velcro^{®} fastener comprises an elongated strip from which a large number of hook devices project, and a device provided with loops that can comprise a strip of textile or non-woven material or a surface layer of the diaper or pants diaper. One problem of using strips that extend downwards past the hips of the user is that the lower edges of the strip can cause irritation of the skin by chafing against the leg of the user when he or she moves.

The present invention aims to solve this problem.

### SUMMARY OF THE INVENTION

This aim is achieved according to the invention through a diaper or pants diaper with a front section, a rear section, and an intermediate crutch section that has a narrower width than the front and rear sections, plus a longitudinal line of symmetry that extends from the middle of the rear edge of the rear section to the middle of the front edge of the front section, where the diaper or pants diaper includes an absorption body that is enclosed between an inner surface layer of material permeable to liquids and an outer surface layer of material impermeable to liquids, whereby the side parts of the front and rear sections can be joined to one another with a refastenable closure by means of a first mechanical join so that the diaper or pants diaper, in the position of use where the side parts are joined to one another in an over-lapping manner, acquires a pants-like configuration with one waist opening and two leg openings characterised in that a band of soft and flexible material, whose one end is permanently attached to the side part of the section of the front or rear section that, when in the position of use, is overlapped by the side part of the other section, is, in the position of use, folded over each edge of the leg opening and attached in a refastenable manner to the outside of the overlapping side part of the mutually joined side parts by means of a second join.

According to one preferred embodiment, each band has a width and placement so that it extends in a cross-sectional direction across the full width of the first mechanical join, and one end of the band is attached to the inner surface layer of the side part of the section of the front or rear section that, in the position of use, is overlapped by the side part of the other section. Alternatively, one end of the band can be attached to the outer surface layer of the side part of the section of the front or rear section that, in the position of use, is overlapped by the side part of the other section.

The diaper can also include a band of soft and flexible material whose one end is permanently attached to the side part of the section of the front or rear section that, in the position of use, is overlapped by the side part of the other section, and that, in the position of use, is folded over each edge of the waist opening and attached by a refastenable closure to the outside of the overlapping side part of the side parts that are joined to one another. Each band can extend from the leg opening to the waist opening of the side part to which the band is permanently attached. The first and second mechanical join can advantageously comprise an interacting hook and loop device. In one advantageous variation, the outer liquid-impermeable surface layer includes an outwards-facing non-woven layer and the band includes a hook device that can be attached to the liquid-impermeable surface layer. The band is preferably made of a non-woven material with a surface weight greater than 30 g/m². Alternatively, the band can be made of non-woven material, at least one side of which is covered with layer of foam plastic. In addition, the band can be thicker at the part of it that extends across the edge of the leg opening when in the position of use of the diaper. In the position of use, each band advantageously extends over the whole length of the associated mechanical join on the inside and/or outside of that join.

### LIST OF FIGURES

The invention will now be described with reference to the following figures, where:
Fig. 1 shows schematically a frontal view of a diaper according to one preferred embodiment of the invention with the inside of the diaper facing the viewer,
Fig. 2 shows a side view of the diaper according to Fig. 1 with the protective band not brought into place,
Fig. 3 shows a side view as in Fig. 2 with the protective band brought into place,
Fig. 4 shows a side view like Fig. 2 of another embodiment of a diaper according to the invention,
Fig. 5 shows a view like Fig. 1 of another embodiment of a diaper according to the invention, and
Fig. 6 shows different variations of attachments and extensions of bands according to the invention.

### DESCRIPTION OF EMBODIMENTS

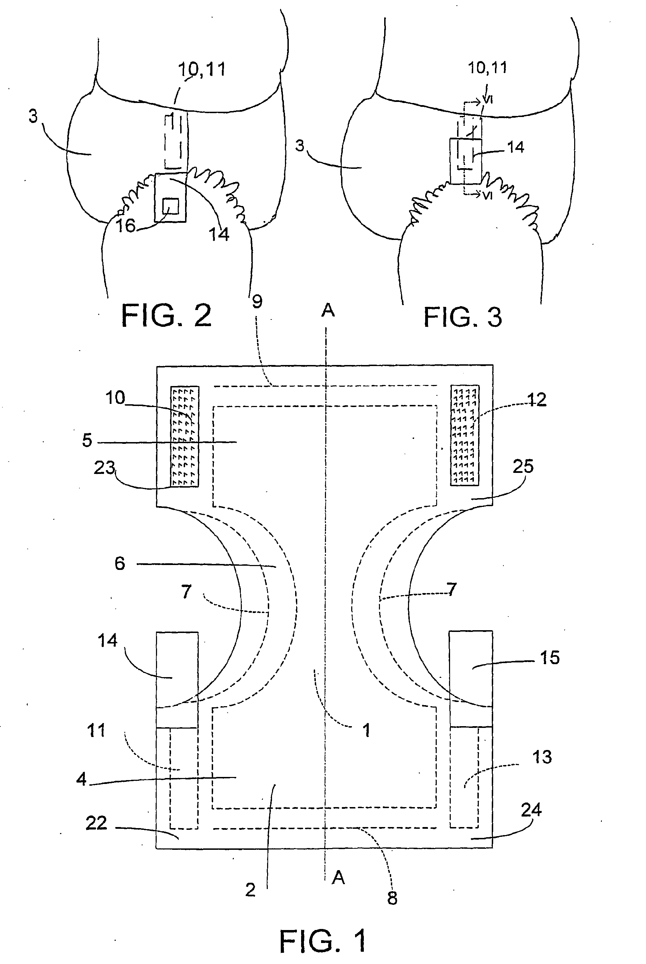

Figures 1-3 show a diaper according to one preferred embodiment of the invention. In the conventional manner, the diaper includes an absorption body 1 enclosed between an inner, liquid-permeable surface layer 2 and an outer, liquid-impermeable surface layer 3. Surface layers 2 and 3 are joined to one another by suitable means, e.g. gluing or thermal sealing, at parts located outside of the absorption body. In addition, the diaper is divided into a front section 4, a rear section 5, and an intermediate crutch section 6 that is narrower than the front and rear sections. The diaper is divided into two symmetrical mirror images by a longitudinal line of symmetry A-A. The diaper has the shape of an hour-glass and elongated elastic threads or elastic bands 7 extend along the tapered sides of the side edges to form the leg elastic. In addition, elastic threads or bands 8 and 9 extend along the front and back edges respectively of the diaper to form the waist elastic. The elastic elements are applied in a pre-tensioned state between the surface layers 2,3 and joined to these. Fig. 1 shows the elastic elements in their pre-tensioned state.

So that the side parts 22,23 respectively 24,25 of the front and rear sections 4,5 of the diaper can be joined to one another, the areas at the long edges of these sections are provided with strips 10,11 respectively 12,13 of attachment devices that interact with one another. In the example shown, strips 10,12 are provided with hook devices while strips 11,13 are provided with loop devices into which the hook devices fit. The strips can comprise devices of the Velcro^{®} type, but also other types of hook and loop devices can be used. Strips 10-13 extend in a lengthwise direction along the greater part of the long edges of the front and rear sections. When the diaper is put in place, the side parts 22,23 respectively 24,25 of the front and rear sections will thus be joined with one another over a large portion of their length, which ensures a good fit for the diaper and allows the fit to be maintained even when the absorption body is filled with discharged liquid. In the embodiment shown, the hook devices are arranged on the strips 10,12 that are attached to the long edge areas of the rear section 5, and the loop devices on the strips 11,13 that are attached to the long edge areas of the front section, but a reversed placement of the hook and loop devices is naturally also possible. The strips 10, 12 with the hook devices are suitably attached to the inner surface layer 2 and the strips 11, 13 with the loop devices suitably attached to the outer surface layer 3 so that the side parts 22,23 respectively 24,25 of the front and rear sections can be joined with one another by overlapping.

In accordance with the invention, a band 14 respectively 15 is attached to the side parts 22,24 of the front section, which bands extend in a cross-sectional direction from the outermost corner of the front section that faces the crutch section and past the corners of the strips 10,11 respectively 12,13 that are turned towards the absorption body. These bands are attached to the inner surface layer 2 at the rear section of the side parts of the front section by a suitable means, e.g. gluing or thermal sealing, and extend in a longitudinal direction some way outside the rear boundaries of these side parts towards the side parts 23,25 of the rear section when the diaper is in the flat condition shown in Fig. 1. The bands 14,15 are made in a soft and flexible material, e.g. non-woven material or a laminate of non-woven material and plastic foam or plastic material.

Fig. 2 shows a schematic side view of the diaper in Fig. 1 as worn by a user. As is evident from this figure, the band 14 extends downwards somewhat at the leg of the user and is provided with a means of attachment 16 so that it can be attached to the outside of the diaper. The means of attachment 16 is of a refastenable type and can comprise an adhesive coating or a piece of material provided with hooks that interacts with the outer non-woven layer of the outer, liquid-impermeable surface layer 3 or with a piece of material provided with loops attached to the outside of the diaper if the outer surface layer does not have an outer non-woven layer. It is pointed out that the means of attachment 16 will only be subjected to minor loadings during the use of the diaper and that its fastening to the outside of the diaper can therefore be weak. Fig. 3 shows a similar view to Fig. 2 but with the band 14 refastened to the outside of the diaper.

The task of the band 14,15 is to prevent the lower edges of the strips 10,11 respectively 12,13 that are joined to one another chafing against the leg of the user when he or she moves. To achieve this function, the band must have a certain thickness (>0.2 mm) and has, therefore, a surface weight greater than 30 g/m², at least within the area that covers the lower edge of the strips 10,11 and 12,13 when these strips are joined to one another. One way of achieving different surface weights for bands 14,15 is to provide them with an extra piece of material within the area that covers the lower edges the strips 10,11 respectively 12,13 when the bands are in place. Such an extra piece of material can naturally be made of a different material than the rest of the band. The band should also be wider than the strips 10-13, although this is not entirely essential.

In one variation, the band 14,15 can be made of a laminate of different materials, e.g. a non-woven material and a foam plastic with a non-woven layer located furthest from the surface layer 2,3. Even a band of textile material can be considered for use.

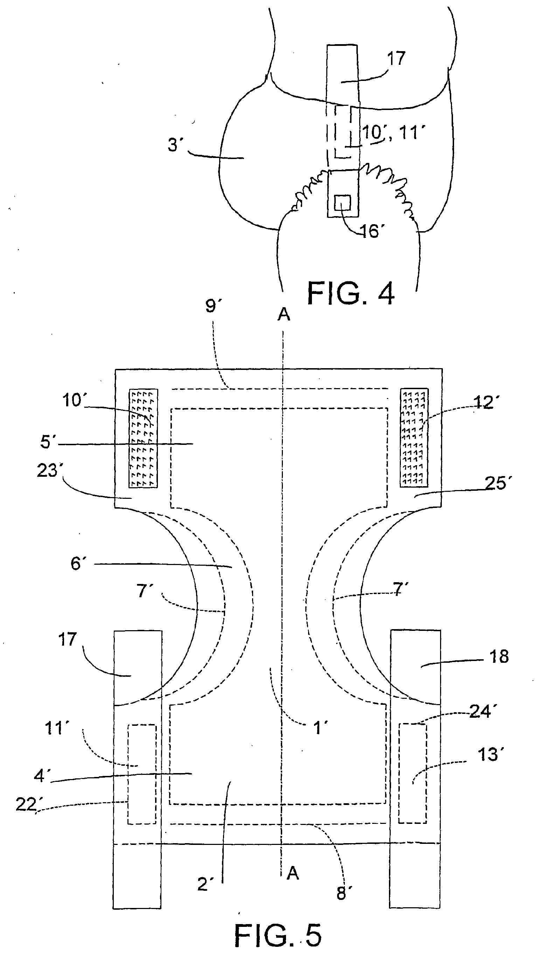

Fig. 4 shows a similar view to Fig. 2 and Fig. 5 a similar view to Fig. 1 of another embodiment of a diaper according to the invention. This embodiment differs from the embodiment shown with reference to Figs. 1-3 only in that the bands 17,18 have a different shape than the bands 14,15. In Fig. 4, components that are the same as the equivalent components in Figs. 1-3 have been given the same reference designation figure with the addition of the prime sign. The bands 17,18 extend along the whole length of the respective long edge area of the front section 4' and also past the front and rear edges of these areas. In addition, bands 17,18 have a length so that the front and rear end parts overlap one another when the bands are folded over the upper and lower edges of the strips 10',11' respectively 12',13' that are attached to one another. The means of attachment 16' is thus designed to be attached to the upper part of the band 17 shown in Fig. 4 instead of to the outer side of the diaper.

The present invention solves in a simple and elegant manner the problem that the edges of strips located at the leg and provided with an interacting means of attachment and joined to one another can give rise to chafing against the leg of the user when he or she moves. The strip edges located at the waist line are not associated with the same risk of chafing when a user moves, but there nevertheless exists a certain risk that the edges can chafe when the user bends forwards, backwards or to the side.

The length of the part of the band 14,15 that extends outside of the lower edge of the diaper in place shown in Fig. 2 need not be greater than that the band can be fastened in the folded-in state to the outside of the diaper. However, for aesthetic reasons, it can be appropriate that the band in its applied position extends from the leg opening to the waistband or only extend a small distance from the leg opening in the direction of the waistband. In addition, it can be appropriate to provide this piece of material with rounded edges. To prevent chafing, the fold of the folded-in band need not be positioned directly adjoining the edges of the strips that are joined with one another; it is sufficient if the edges of the strips are covered by the band after folding inwards.

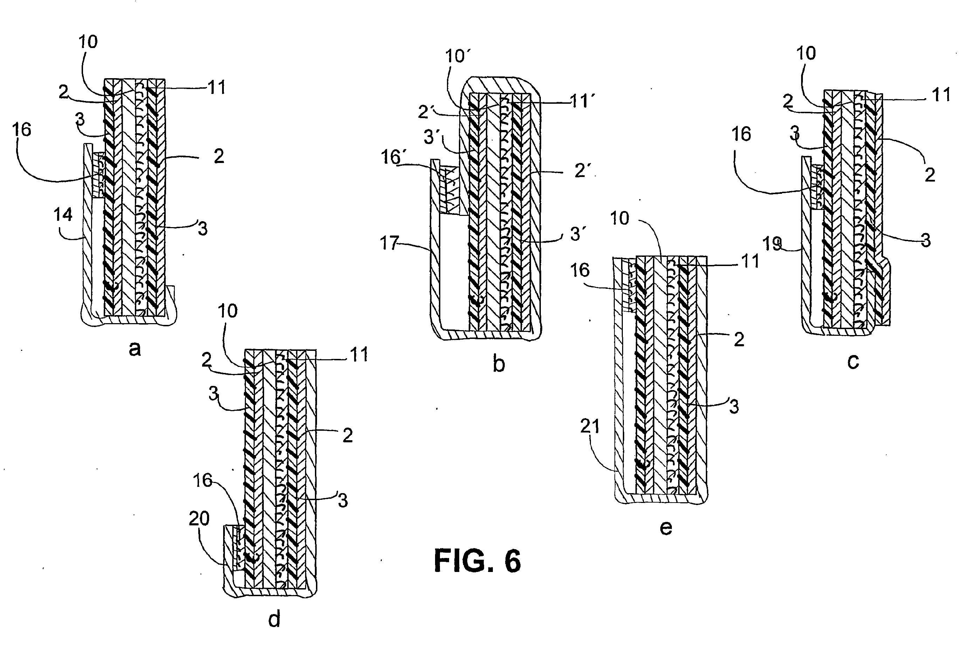

Fig. 6a shows a cross-sectional view along the line VI-VI in Fig. 3 and Fig. 6b shows an equivalent view through the diaper according to the embodiment shown in Figs. 4 and 5. Figs. 6c-6e show equivalent views of other embodiments of the diaper according to the invention. In all figures except Fig. 6c, the bands 14,17, 20 and 21 are attached to the inner surface layer 2 of the overlapping side part of the diaper, while the band 19 in the embodiment shown in Fig. 6c is attached to the outer surface layer 3 of the overlapping side part of the diaper. Naturally, even the bands in Figs. 6a,b,d and e can instead be attached to the outer surface layer of the overlapping side part. As is evident from the figures, the bands can also have different extensions in height, but it is preferable if they either extend over the first mechanical join 10,11 or only past the upper or lower edge of this join, as shown schematically in Fig. 6e respectively 6d. In addition, the attached end of the band can extend in height along the whole length of the join 10,11 as shown in Figs. 6b,d and e, or only over a part of this, as shown in Figs. 6a and c.

The inner, liquid-permeable surface layer 2 of the diaper suitably comprises non-woven material or a non-woven laminate. Also all other materials used for the inner, liquid-permeable surface layer of diapers and similar absorbing articles can naturally be used, such as perforated plastic films.

The outer liquid-impermeable surface layer is preferably made of a laminate of vapour-permeable plastic film and a non-woven material, preferably one that can act as loops for the hooks of the chosen strip provided with hooks, whereby separate strips 11,13 provided with loops are no longer needed. All other materials used for the liquid-impermeable surface layer of diapers and similar absorbing articles can naturally also be used.

The absorption body is preferably made of one or several layers of cellulose stuffing with or without the addition of particles of so-called super-absorbent material. Other types of fibre, such as binder fibre, can be mixed in the cellulose stuffing. In addition, layers with good permeability capabilities, e.g. hydrophobic wadding, can be included in the absorption body. All other material that is used in absorption body for absorbing articles, for example foam plastic, can naturally also be used.

In the described embodiments, the absorption body, like the outer covering of the diaper, has the shape of an hour-glass, but it is naturally possible to give the absorption body another shape, for example, a rectangular form.

The described embodiments show diapers, but the invention can also be applied to pants diapers that can be opened, whereby the strips provided with attachment devices are joined to one another and the bands that cover the edges of the strips are attached to the outside of the pants diapers in conjunction with manufacture of the pants diapers and are supplied in an assembled state. The side parts, i.e. those parts of the outer covering that extend in a sideways direction outside of the absorption body, are preferably elasticised for a pants diaper. Also diapers can be provided with elasticised side panels.

The described embodiments can naturally be modified within the scope of the invention. For example, the corners of the strips can be rounded to further reduce the risk of chafing. The outer surface layer need not be liquid-impermeable over the whole of its surface but can conceivably have that part of the outer surface that does not cover the absorption body perforated or consisting of material that is very permeable to air, e.g. a net material. If the diaper or pants diaper has elasticised side panels, these can be made of separate panels of elasticised material or elasticised parts of one or both surface layers. The mechanical first join need not comprise hook and loop devices but can be composed of other types of mechanical join, such as buttons and button holes, snap-in fastenings, etc. In addition, the band need not comprise separate pieces of material but can be composed of surface layer parts that remain after punching out the leg openings. The invention shall thus be limited only by the contents in the attached claims.

## Claims

1. Diaper or pants diaper with a front section (4;4'), a rear section (5;5'), and an intermediate crutch section (6;6') that has a narrower width than the front and rear sections plus a longitudinal line of symmetry (A-A) that extends from the middle of the rear edge of the rear section to the middle of the front edge of the front section, wherein the diaper or pants diaper includes an absorption body (1;1') that is enclosed between a inner surface layer (2;2') of material permeable to liquids and an outer surface layer (3;3') of material impermeable to liquids, whereby the side parts (22,23 respectively 24,25; 22',23' respectively 24',25'), of the front and rear sections can be joined to one another by a refastenable closure by means of a first mechanical join (10,11 respectively 12,13;10',11' respectively 12',13') so that the diaper or pants diaper, in the position of use where the side parts are joined to one another in an over-lapping manner, acquires a pants-like configuration with one waist opening and two leg openings **characterised in that** a band (14,15;17,18) of soft and flexible material, whose one end is permanently attached to the side part (22,24; respectively 22',24') of that section of the front or rear section (4,5) that, when in the position of use, is overlapped by the side part (23,25; respectively 23',25') of the other section, is, in the position of use, folded over each edge of the leg opening and refastenable to the outside of the overlapping side part (23,25;23',25') of the mutually joined side parts (22,23 respectively 24,25;22',23' respectively 24',25') by means of a second join (16).

2. Diaper or pants diaper according to claim 1 **characterised in that** each band (14,15;17,18) has a width and placement such that in a cross-sectional direction it extends over the whole width of the first mechanical join (10,11 respectively 12,13;10',11' respectively 12',13').

3. Diaper or pants diaper according to claim 1 or 2 **characterised in that** one end of the band (14,15;17,18) is attached to the inner surface layer (2;2') of the side part (22,24;22',24') of the section of the front or rear section (4,5) that, in the position of use, is overlapped by the side part (23,25;23',25') of the other section.

4. Diaper or pants diaper according to claim 1 or 2 **characterised in that** one end of the band (14,15;17,18) is attached to the outer surface layer (3;3') of the side part (22,24;22',24') of the section of the front or rear section (4,5) that, in the position of use, is overlapped by the side part (23,25;23',25') of the other section.

5. Diaper or pants diaper according to any of claims 1-4 **characterised in that** a band (17,18) of soft and flexible material, whose one end is permanently attached to the side part (22',24') of the section of the front or rear section (4',5') that, in the position of use, is overlapped by the side part (23',25') of the other section, is, in the position of use, folded over each edge of the waist opening and refastenable to the outside of the overlapping side part (23.',25') of the mutually joined side parts.

6. Diaper or pants diaper according to claim 5 **characterised in that** each band (17,18) extends from the leg opening to the waist opening of the side part (22',24') to which the band is permanently attached.

7. Diaper or pants diaper according to any of claims 1-6 **characterised in that** the first mechanical join (10,11 respectively 12,13;10',11' respectively 12',13') comprises interacting hook and loop devices.

8. Diaper or pants diaper according to any of claims 1-7 **characterised in that** the second mechanical join (16;16') comprises interacting hook and loop devices.

9. Diaper or pants diaper according to any of claims 1-8 **characterised in that** the liquid-impermeable surface layer includes an outwards facing non-woven layer and that the band includes hook devices than can be attached to the liquid-impermeable surface layer.

10. Diaper or pants diaper according to any of claims 1-9 **characterised in that** the band (14,15;17,18) is made of non-woven material with a surface weight greater than 30 g/m².

11. Diaper or pants diaper according to any of claims 1-10 **characterised in that** the band (14,15;17,18) is made of non-woven material, one side of which is at least partly covered with a layer of foam plastic.

12. Diaper or pants diaper according to any of claims 1-11 **characterised in that** the band (14,15) is thicker in the part of it that, in the position of use, extends over the edge of the leg opening.

13. Diaper or pants diaper according to any of claims 1-12 **characterised in that** each band (17,18), in the position of use, extends over the whole length of the associated first mechanical join (10,11 respectively 12,13) on the inside and/or outside of this join.

## Patentansprüche

1. Windel oder Höschenwindel mit einem frontseitigen Abschnitt (4; 4'), einem rückseitigen Abschnitt (5; 5') und einem dazwischen liegenden Schrittabschnitt (6; 6'), der eine schmalere Breite als der frontseitige und der rückseitige Abschnitt und eine Symmetrieachse (A-A) in Längsrichtung, die sich von der Mitte der rückseitigen Kante des rückseitigen Abschnitts zu der Mitte der frontseitigen Kante des frontseitigen Abschnitts erstreckt, aufweist, wobei die Windel oder Höschenwindel einen Absorptionskörper (1; 1') aufweist, der zwischen einer inneren Oberflächenlage (2; 2') eines flüssigkeitsdurchlässigen Materials und einer äußeren Oberflächenlage (3; 3') eines flüssigkeitsundurchlässigen Materials eingeschlossen ist, wodurch die Seitenteile (22, 23 bzw. 24, 25; 22', 23' bzw. 24', 25') des frontseitigen und rückseitigen Abschnitts miteinander durch einen wieder verschließbaren Verschluss mittels einer ersten mechanischen Verbindung (10, 11 bzw. 12, 13; 10', 11' bzw. 12', 13') verbunden werden können, so dass die Windel oder Höschenwindel in der Benutzungsposition, in der die Seitenteile miteinander auf überlappende Weise verbunden sind, eine hosenförmige Anordnung mit einer Taillenöffnung und zwei Beinöffnungen annimmt, **dadurch gekennzeichnet, dass** ein Band (14, 15; 17, 18) eines weichen und flexiblen Materials, dessen eines Ende permanent an dem Seitenteil (22, 24; bzw. 22', 24') dieses Abschnitts des frontseitigen oder rückseitigen Abschnitts (4, 5) angebracht ist, der, wenn er in der Benutzungsposition ist, von dem Seitenteil (23, 25; bzw. 23', 25') des anderen Abschnitts überlappt wird, in der Benutzungsposition über jede Kante der Beinöffnung gefaltet und an der Außenseite des überlappenden Seitenteils (23, 25; 23', 25') der miteinander verbundenen Seitenteile (22, 23, bzw. 24, 25; 22', 23' bzw. 24', 25') mittels einer zweiten Verbindung (16) wieder befestigbar ist.

2. Windel oder Höschenwindel nach Anspruch 1, **dadurch gekennzeichnet, dass** jedes Band (14, 15; 17, 18) eine solche Breite und Platzierung aufweist, dass es sich in einer Querschnittsrichtung über die gesamte Breite der ersten mechanischen Verbindung (10, 11 bzw. 12, 13; 10', 11' bzw. 12', 13') erstreckt.

3. Windel oder Höschenwindel nach Anspruch 1 oder 2, **dadurch gekennzeichnet, dass** ein Ende des Bands (14, 15; 17, 18) an der inneren Oberflächenlage (2; 2') des Seitenteils (22, 24; 22', 24') des Abschnitts des frontseitigen oder rückseitigen Abschnitts (4, 5) angebracht ist, der in der Benutzungsposition von dem Seitenteil (23, 25; 23', 25') des anderen Abschnitts überlappt wird.

4. Windel oder Höschenwindel nach Anspruch 1 oder 2, **dadurch gekennzeichnet, dass** ein Ende des Bands (14, 15; 17, 18) an der äußeren Oberflächenlage (3; 3') des Seitenteils (22, 24; 22', 24') des Abschnitts des frontseitigen oder rückseitigen Abschnitts (4, 5) angebracht ist, der in der Benutzungsposition von dem Seitenteil (23, 25; 23', 25') des anderen Abschnitts überlappt wird.

5. Windel oder Höschenwindel nach einem der Ansprüche 1-4, **dadurch gekennzeichnet, dass** ein Band (17, 18) eines weichen und flexiblen Materials, dessen eines Ende permanent an dem Seitenteil (22', 24') des Abschnitts des frontseitigen oder rückseitigen Abschnitts (4', 5') angebracht ist, der in der Benutzungsposition von dem Seitenteil (23', 25') des anderen Abschnitts überlappt wird, in der Benutzungsposition über jede Kante der Taillenöffnung gefaltet ist und an der Außenseite des überlappenden Seitenteils (23', 25') der miteinander verbundenen Seitenteile wieder befestigbar ist.

6. Windel oder Höschenwindel nach Anspruch 5, **dadurch gekennzeichnet, dass** sich jedes Band (17, 18) von der Beinöffnung zu der Taillenöffnung des Seitenteils (22', 24'), an dem das Band permanent angebracht ist, erstreckt.

7. Windel oder Höschenwindel nach einem der Ansprüche 1-6, **dadurch gekennzeichnet, dass** die erste mechanische Verbindung (10, 11 bzw. 12, 13; 10', 11' bzw. 12', 13') miteinander zusammenwirkende Klettverschlussvorrichtungen umfasst.

8. Windel oder Höschenwindel nach einem der Ansprüche 1-7, **dadurch gekennzeichnet, dass** die zweite mechanische Verbindung (16; 16') miteinander zusammenwirkende Klettverschlussvorrichtungen umfasst.

9. Windel oder Höschenwindel nach einem der Ansprüche 1-8, **dadurch gekennzeichnet, dass** die flüssigkeitsundurchlässige Oberflächenlage eine nach außen gerichtete Vlieslage aufweist und dass das Band Hakenvorrichtungen aufweist, die an der flüssigkeitsundurchlässigen Oberflächenlage befestigt werden können.

10. Windel oder Höschenwindel nach einem der Ansprüche 1-9, **dadurch gekennzeichnet, dass** das Band (14, 15; 17, 18) aus Vliesmaterial mit einem Oberflächengewicht von mehr als 30 g/m² hergestellt ist.

11. Windel oder Höschenwindel nach einem der Ansprüche 1-10, **dadurch gekennzeichnet, dass** das Band (14, 15; 17, 18) aus Vliesmaterial gemacht ist, dessen eine Seite zumindest teilweise mit einer Lage Kunststoffschaum bedeckt ist.

12. Windel oder Höschenwindel nach einem der Ansprüche 1-11, **dadurch gekennzeichnet, dass** das Band (14, 15) in seinem Teil, der sich in der Benutzungsposition über die Kante der Beinöffnung erstreckt, dicker ist.

13. Windel oder Höschenwindel nach einem der Ansprüche 1-12, **dadurch gekennzeichnet, dass** sich jedes Band (17, 18) in der Benutzungsposition über die gesamte Länge der mit ihm verbundenen ersten mechanischen Verbindung (10, 11 bzw. 12, 13) an der Innenseite und/oder Außenseite dieser Verbindung erstreckt.

## Revendications

1. Couche ou couche-culotte comportant une partie avant (4 ; 4'), une partie arrière (5 ; 5'), et une partie entrejambe intermédiaire (6 ; 6') qui a une largeur inférieure à celle de la partie avant et de la partie arrière, et en outre une ligne de symétrie longitudinale (A-A) qui s'étend depuis le milieu du bord arrière de la partie arrière jusqu'au milieu du bord avant de la partie avant, la couche, ou la couche-culotte, comprenant un corps absorbant (1 ; 1') qui est enfermé entre une couche de surface intérieure (2 ; 2') constituée d'un matériau perméable aux liquides et une couche de surface extérieure (3 ; 3') constituée d'un matériau imperméable aux liquides, les zones latérales (22, 23 respectivement 24, 25 ; 22', 23' et respectivement 24', 25') des parties avant et arrière pouvant être reliées l'une à l'autre par un dispositif de fermeture rattachable au moyen d'une première jonction mécanique (10, 11 respectivement 12, 13 ; 10', 11' respectivement 12', 13') de telle manière que la couche, ou la couche-culotte, lorsqu'elle se trouve en position d'utilisation dans laquelle les zones latérales sont reliées l'une à l'autre en se chevauchant prend une configuration analogue à celle d'une culotte comportant une ouverture pour la taille et deux ouvertures pour les jambes, **caractérisée en ce qu'**une bande (14, 15 ; 17, 18) de matériau doux et souple, dont une des extrémités est fixée de façon permanente à la zone latérale (22, 24 ; respectivement 22', 24') de celle des parties avant et arrière (4, 5) qui en position d'utilisation est chevauchée par la zone latérale (23, 25 ; respectivement 23', 25') de l'autre partie, est, en position d'utilisation, repliée sur chaque bord de l'ouverture pour la jambe et peut être rattachée sur l'extérieur de la zone latérale chevauchante 23, 25 ; 23', 25') des zones latérales mutuellement reliées (22, 23 respectivement 24, 25 ; 22', 23' respectivement 24', 25') au moyen d'une deuxième jonction (16).

2. Couche ou couche-culotte selon la revendication 1, **caractérisée en ce que** chaque bande (14, 15 ; 17, 18) a une largeur telle, et un positionnement tel, qu'elle s'étend dans une direction de coupe sur toute la largeur de la première jonction mécanique (10, 11 respectivement 12, 13 ; 10', 11' ; respectivement 12', 13').

3. Couche ou couche-culotte selon la revendication 1 ou 2, **caractérisée en ce qu'**une des extrémités de la bande (14, 15 ; 17, 18) est fixée à la couche de surface intérieure (2, 2') de la zone latérale (22, 24 ; 22', 24') de celle des parties avant et arrière (4, 5) qui, en position d'utilisation, est chevauchée par la zone latérale (23, 25 ; 23', 25') de l'autre partie.

4. Couche ou couche-culotte selon la revendication 1 ou 2, **caractérisée en ce qu'**une des extrémités de la bande (14, 15 ; 17, 18) est fixée à la couche de surface extérieure (3, 3') de la zone latérale 22, 24 ; 22', 24') de celle des parties avant ou arrière (4, 5) qui, en position d'utilisation, est chevauchée par la zone latérale (23, 25 ; 23', 25') de l'autre partie.

5. Couche ou couche-culotte selon l'une quelconque des revendications 1 à 4, **caractérisée en ce qu'**une bande (17, 18) de matériau doux et souple, dont une des extrémités est fixée de façon permanente à la zone latérale (22', 24') de celle des parties avant et arrière (4', 5') qui, en position d'utilisation, est chevauchée par la zone latérale (23', 25') de l'autre partie, est, en position d'utilisation, repliée sur chaque bord de l'ouverture pour la taille et peut être rattachée sur l'extérieur de celle des zones latérales (23', 25') mutuellement reliées qui est chevauchante.

6. Couche ou couche-culotte selon la revendication 5, **caractérisée en ce que** chaque bande (17, 18) s'étend depuis l'ouverture pour la jambe jusqu'à l'ouverture pour la taille de la zone latérale (22', 24') à laquelle la bande est fixée de façon permanente.

7. Couche ou couche-culotte selon l'une quelconque des revendications 1 à 6, **caractérisée en ce que** la première jonction mécanique (10, 11 respectivement 12, 13 ; 10', 11' et respectivement 12', 13') comporte des dispositifs à boucle et crochet qui coopèrent l'un avec l'autre.

8. Couche ou couche-culotte selon l'une quelconque des revendications 1 à 7, **caractérisée en ce que** la deuxième jonction mécanique (16, 16') comporte des dispositifs à boucle et à crochet qui coopèrent l'un avec l'autre.

9. Couche ou couche-culotte selon l'une quelconque des revendications 1 à 8, **caractérisée en ce que** la couche de surface imperméable aux liquides comporte une couche de non-tissé orientée vers l'extérieur et **en ce que** la bande comporte des dispositifs à crochet qui peuvent être fixés à la couche de surface imperméable aux liquides.

10. Couche ou couche-culotte selon l'une quelconque des revendications 1 à 9, **caractérisée en ce que** la bande (14, 15 ; 17, 18) est constituée d'un matériau non tissé doté d'une masse surfacique supérieure à 30 g/m².

11. Couche ou couche-culotte selon l'une quelconque des revendications 1 à 10, **caractérisée en ce que** la bande (14, 15 ; 17, 18) est constituée d'un matériau non tissé dont un côté est au moins partiellement recouvert d'une couche de matière plastique mousse.

12. Couche ou couche-culotte selon l'une quelconque des revendications 1 à 11, **caractérisée en ce que** la bande (14, 15) est plus épaisse dans sa zone qui, en position d'utilisation, s'étend sur le bord de l'ouverture pour la jambe.

13. Couche ou couche-culotte selon l'une quelconque des revendications 1 à 12, **caractérisée en ce que** chaque bande (17, 18) s'étend, en position d'utilisation sur toute la longueur de la première jonction mécanique associée (10, 11 respectivement 12, 13) sur l'intérieur et/ou sur l'extérieur de cette jonction.
